# EUROPEAN PATENT APPLICATION

(11) **EP 3 872 670 A1**
(43) Date of publication of application: **01.09.2021**
(21) Application number: 21159389.2
(22) Date of filing: 25.02.2021
(51) Int. Cl.: G06F 21/64, H04L 9/32, H04L 29/06, H04L 29/08

(54) **METHOD AND APPARATUS FOR SHARING DATA BASED ON BLOCKCHAIN NETWORK, DEVICE, AND MEDIUM**

(30) Priority: 26.02.2020 CN 202010119729
(71) Applicant: BAIDU ONLINE NETWORK TECHNOLOGY (BEIJING) CO., LTD., Beijing 100085 (CN)
(72) Inventor: FAN, Wei, Haidian District, Beijing 100085 (CN); WANG, Yucao, Haidian District, Beijing 100085 (CN); YU, Yanan, Haidian District, Beijing 100085 (CN)
(74) Representative: Zimmermann, Tankred Klaus

(57) **Abstract**

The disclosure provides a method for sharing data based on a blockchain network, a device and a medium. In response to a sharing transaction request including target data of a data provider, a chaining operation is performed (S101) on the target data for storing the target data. A sharing smart contract is called (S102) to generate a sharing certificate including an identifier of a source organization, an identifier of a target organization and a storage identifier of the target data, and the chaining operation is performed on the sharing certificate for storing the sharing certificate. The target data is shared (S103) with the target organization based on the sharing certificate.

## Description

### FIELD

The disclosure relates to a field of computer technologies and particularly to a field of blockchain technologies, and particularly relates to a method and an apparatus for sharing data based on a blockchain network, a device and a medium.

### BACKGROUND

With continuous development of the information age, data exchange between different organizations has gradually increased.

In the related art, when data sharing needs to be performed between a source organization and a target organization in an organization union containing multiple organizations, the target organization obtains authorization by issuing a paper sharing certificate and telephone confirmation, and then transmits data between the source organization and the target organization through a centralized server.

With the above way, the data sharing is performed between the organizations, and the authorization is generally granted by means of issuing the paper certificate and the telephone confirmation. Therefore, the data sharing efficiency is low, and it is difficult to ensure the accuracy of the data in a sharing certificate.

### SUMMARY

Embodiments of the disclosure provide a method and an apparatus for sharing data based on a blockchain network, a device and a medium, thereby sharing target data between a source organization and a target organization contained in an organization union, and improving a efficiency for sharing the target data.

In a first aspect, embodiments of the disclosure provide a method for sharing data based on a blockchain network. The method is executed by a blockchain node in the blockchain network. The method includes: in response to a sharing transaction request including target data of a data provider, performing a chaining operation on the target data for storing the target data; calling a sharing smart contract to generate a sharing certificate including an identifier of a source organization, an identifier of a target organization and a storage identifier of the target data, and performing the chaining operation on the sharing certificate for storing the sharing certificate; and sharing the target data with the target organization based on the sharing certificate.

With embodiments of the disclosure, in response to the sharing transaction request including the target data of the data provider, the chaining operation is performed on the target data for storing the target data. The sharing smart contract is called to generate the sharing certificate including the identifier of the source organization, the identifier of the target organization and the storage identifier of the target data. The chaining operation is performed on the sharing certificate for storing the sharing certificate. The target data is shared with the target organization based on the sharing certificate. With the above technical solution, the chaining operation is performed on the sharing certificate for storing the sharing certificate by calling the sharing smart contract, such that the target organization obtains the target data based on the sharing certificate stored on the chain, thereby realizing data sharing of the target data between the source organization and the target organization, without issuing or confirming a paper certificate for authorization, improving the data sharing efficiency. Meanwhile, the sharing certificate is generated by calling the sharing smart contract, thereby reducing the impact of human operations on the accuracy of the sharing certificate, and improving the accuracy of the sharing certificate.

In some embodiments, sharing the target data with the target organization based on the sharing certificate includes: obtaining the target data based on the storage identifier of the target data and the identifier of the source organization in the sharing certificate and feeding the target data back to the target organization, in response to a data obtaining transaction request including the sharing certificate and sent by the target organization.

With an alternative implementation of the disclosure, the operation "sharing the target data" is refined to the operation "sharing the target data with the target organization in response to a data obtaining transaction request including the sharing certificate and sent by the target organization", thereby improving a sharing way of the target data.

In some embodiments, before responding to the data obtaining transaction request including the sharing certificate and sent by the target organization, the method also includes: sending a storage identifier of the sharing certificate to a to-be-processed queue of the target organization, to allow the target organization to obtain the sharing certificate based on the storage identifier of the sharing certificate in the to-be-processed queue.

With an alternative implementation of the disclosure, the storage identifier of the sharing certificate is sent to the to-be-processed queue of the target organization before responding to the data obtaining transaction request, thereby providing convenience for the target organization to obtain sharing data.

In some embodiments, the method also includes: calling a queue access smart contract to obtain the storage identifier of the sharing certificate from the to-be-processed queue of the target organization in response to a queue access transaction request initiated by the target organization; and obtaining the sharing certificate based on the storage identifier of the sharing certificate, and feeding the sharing certificate back to the target organization.

With an alternative implementation of the disclosure, the storage identifier of the sharing certificate is obtained from the to-be-processed queue by responding to the queue access transaction request initiated by the target organization, thereby providing convenience for obtaining the sharing certificate.

In some embodiments, the sharing certificate also includes a digital digest of the target data.

Correspondingly, obtaining the target data based on the storage identifier of the target data and the identifier of the source organization in the sharing certificate includes: searching for the target data based on the storage identifier of the target data and the identifier of the source organization in the sharing certificate; performing verification on the target data found based on the digital digest; and obtaining the target data and feeding the target data back to the target organization when the target data passes the verification.

With an alternative implementation of the disclosure, by adding the digital digest of the target data to the sharing certificate, the accuracy of the target data is verified based on the data digest during obtaining the target data.

In some embodiments, performing the chaining operation on the sharing certificate for storing the sharing certificate includes: sending the sharing certificate to the source organization, to allow the source organization to sign the sharing certificate through a private key of the source organization; and obtaining a digital signature of the sharing certificate from the source organization, associating the digital signature with the sharing certificate, and performing the chaining operation on the digital signature and the sharing certificate for storing the digital signature and the sharing certificate.

Correspondingly, obtaining the target data based on the storage identifier of the target data and the identifier of the source organization in the sharing certificate and feeding the target data back to the target organization includes: performing verification on the digital signature from the source organization based on the identifier of the source organization in the sharing certificate; and obtaining the target data based on the storage identifier of the target data and the identifier of the source organization in the sharing certificate and feeding the target data back to the target organization when the digital signature passes the verification.

With an alternative implementation of the disclosure, the source organization performs the digital signature on the sharing certificate when the chaining operation is performed on the sharing certificate for storing the sharing certificate, such that the target organization may verify a validity of the sharing certificate based on the identifier of the source organization in the sharing certificate when obtaining the sharing certificate.

In some embodiments, performing the chaining operation on the target data for storing the target data includes: calling a data storage interface of the sharing smart contract to perform the chaining operation on the target data for storing the target data.

Calling the sharing smart contract to generate the sharing certificate including the identifier of the source organization, the identifier of the target organization and the storage identifier of the target data and performing the chaining operation on the sharing certificate for storing the sharing certificate includes: calling a sharing certificate generating interface of the sharing smart contract to generate the sharing certificate including the identifier of the source organization, the identifier of the target organization and the storage identifier of the target data and performing the chaining operation on the sharing certificate for storing the sharing certificate.

With an alternative implementation of the disclosure, the operation that the chaining operation is performed on the target data for storing the target data, the sharing certificate is generated, and the chaining operation is performed on the sharing certificate for storing the sharing certificate is implemented by calling a data interface of the sharing smart contract, thereby providing convenience for reading the target data and the sharing certificate.

In some embodiments, the target data is obtained by encrypting original content based on a key of the data provider and a key of the target organization.

With an alternative implementation of the disclosure, the target data may be obtained from the original content by the key of the data provider and the key of the target organization, which may provide authorization for the target organization during sharing the target data, prevent the target data from leaking to organizations other than the source organization and the target organization, and improve the security of the target data.

In some embodiments, the organization is a hospital, the target data of the data provider is diagnosis and treatment data of patients, and the sharing certificate is a referral certificate.

With an alternative implementation of the disclosure, by refining the organization, the target data and the sharing certificate, the data sharing operation is applied to a scene where a patient is transferred from a source hospital to a target hospital.

In a second aspect, embodiments of the disclosure also provide a method for sharing data based on a blockchain network. The method is executed by a source organization. The method includes: obtaining target data of a data provider; and initiating a sharing transaction request including the target data, to allow a blockchain node to perform a chaining operation on the target data for storing the target data, generate a sharing certificate including an identifier of the source organization, an identifier of a target organization and a storage identifier of the target data by calling a sharing smart contract, and perform the chaining operation on the sharing certificate for storing the sharing certificate.

The sharing certificate is configured to indicate the target organization to share the target data.

In third aspect, embodiments of the disclosure provide a method for sharing data based on a blockchain network. The method is executed by a target organization. The method includes: obtaining a sharing certificate including an identifier of a source organization, an identifier of the target organization and a storage identifier of target data, in which the sharing certificate is generated by a blockchain node after performing a chaining operation on the target data for storing the target data and calling a sharing smart contract in response to a sharing transaction request comprising the target data; and initiating a data obtaining transaction request including the sharing certificate, to allow the blockchain node to share the target data with the target organization based on the sharing certificate.

In a fourth aspect, embodiments of the disclosure also provide an apparatus for sharing data based on a blockchain network. The apparatus is integrated into a blockchain node. The apparatus includes: a target data chaining module, a sharing certificate chaining module, and a target data sharing module. The target data chaining module is configured to, in response to a sharing transaction request including target data of a data provider, perform a chaining operation on the target data for storing the target data. The sharing certificate chaining module is configured to call a sharing smart contract, to generate a sharing certificate including an identifier of a source organization, an identifier of a target organization and a storage identifier of the target data, and perform the chaining operation on the sharing certificate for storing the sharing certificate. The target data sharing module is configured to share the target data with the target organization based on the sharing certificate.

In a fifth aspect, embodiments of the disclosure also provide an apparatus for sharing data based on a blockchain network. The apparatus is integrated into a source organization. The apparatus includes: a target data obtaining module, and a sharing transaction request initiating module. The target data obtaining module is configured to obtain target data of a data provider. The sharing transaction request initiating module is configured to initiate a sharing transaction request including the target data, to allow a blockchain node to perform a chaining operation on the target data for storing the target data, generate a sharing certificate including an identifier of a source organization, an identifier of a target organization and a storage identifier of the target data by calling a sharing smart contract, and perform a chaining operation on the sharing certificate for storing the sharing certificate. The sharing certificate is configured to indicate the target organization to share the target data.

In a sixth aspect, embodiments of the disclosure also provide an apparatus for sharing data based on a blockchain network. The apparatus is integrated into a target organization. The apparatus includes: a sharing certificate obtaining module and a transaction request initiating module. The sharing certificate obtaining module is configured to obtain a sharing certificate including an identifier of a source organization, an identifier of the target organization and a storage identifier of target data. The sharing certificate is generated by a blockchain node after performing a chaining operation on the target data for storing the target data and calling a sharing smart contract in response to a sharing transaction request comprising the target data. The transaction request initiating module is configured to initiate a data obtaining transaction request including the sharing certificate, to allow the blockchain node to share the target data with the target organization based on the sharing certificate.

In a seventh aspect, embodiments of the disclosure also provide an electronic device. The electronic device includes: at least one processor and a memory. The memory is communicatively coupled to the at least one processor. The memory is configured to store instructions executable by the at least one processor. When the instructions are executed by the at least one processor, the at least one processor is caused to execute the method for sharing the data based on the blockchain network according to the first aspect, the second aspect, and the third aspect.

In an eighth aspect, embodiments of the disclosure also provide a non-transitory computer readable storage medium having computer instructions stored thereon. The computer instructions are configured to cause a computer to execute the method for sharing the data based on the blockchain network according to the first aspect, the second aspect, and the third aspect.

Other effects of the above alternative modes will be explained in the following with specific embodiments.

### BRIEF DESCRIPTION OF THE DRAWINGS

The accompanying drawings are used for better understanding the solution and do not constitute a limitation of the disclosure.
FIG. 1 is a flowchart illustrating a method for sharing data based on a blockchain network according to embodiments of the disclosure.
FIG. 2 is a flowchart illustrating a method for sharing data based on a blockchain network according to embodiments of the disclosure.
FIG. 3 is a flowchart illustrating a method for sharing data based on a blockchain network according to embodiments of the disclosure.
FIG. 4 is a flowchart illustrating a method for sharing data based on a blockchain network according to embodiments of the disclosure.
FIG. 5 is a flowchart illustrating a method for sharing data based on a blockchain network according to embodiments of the disclosure.
FIG. 6 is a block diagram illustrating an apparatus for sharing data based on a blockchain network according to embodiments of the disclosure.
FIG. 7 is a block diagram illustrating an apparatus for sharing data based on a blockchain network according to embodiments of the disclosure.
FIG. 8 is a block diagram illustrating an apparatus for sharing data based on a blockchain network according to embodiments of the disclosure.
FIG. 9 is a block diagram illustrating an electronic device capable of implementing a method for sharing data based on a blockchain network according to embodiments of the disclosure.

### DETAILED DESCRIPTION

Description will be made below to exemplary embodiments of the disclosure with reference to accompanying drawings, which includes various details of embodiments of the disclosure to facilitate understanding and should be regarded as merely examples. Therefore, it should be recognized by the skilled in the art that various changes and modifications may be made to the embodiments described herein without departing from the scope and spirit of the disclosure. Meanwhile, for clarity and conciseness, descriptions for well-known functions and structures are omitted in the following description.

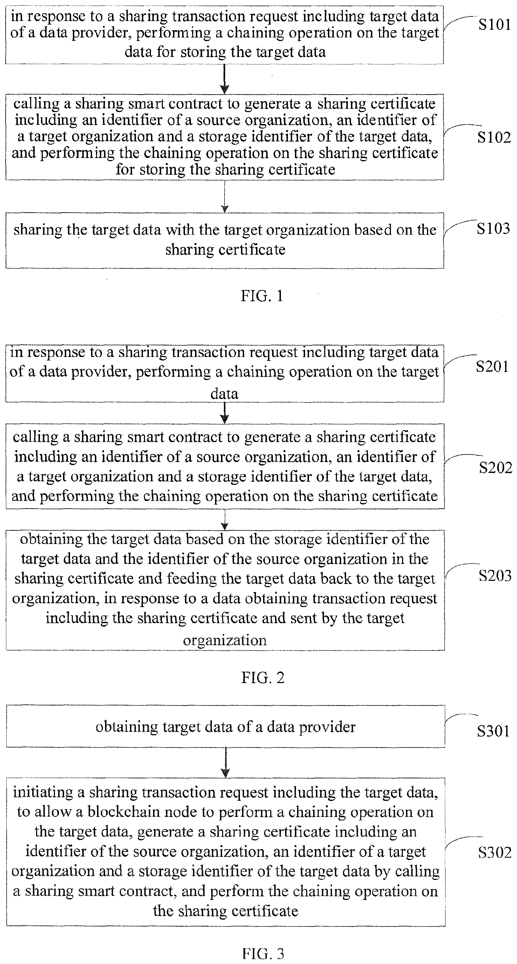

FIG. 1 is a flowchart illustrating a method for sharing data based on a blockchain network according to embodiments of the disclosure. Embodiments of the disclosure may be applicable to a condition where target data is shared between a source organization and a target organization in an organization union containing multiple organizations. The method may be executed by an apparatus for sharing data based on a blockchain network. The apparatus may be implemented by software and/or hardware and integrated in an electronic device carrying a blockchain node.

The method for sharing data based on a blockchain network as illustrated in FIG. 1 may include the following.

At block S101, in response to a sharing transaction request including target data of a data provider, a chaining operation is performed on the target data for storing the target data.

In some embodiments, the sharing transaction request may be initiated by the data provider to the blockchain network based on the target data of the data provider, such that the target data may be shared between the source organization and the target organization in the organization union. In some embodiments, the source organization may initiate the sharing transaction request to the blockchain network based on the target data of the data provider.

The target data may be obtained by the source organization from the data provider in an offline obtaining way, or the target data may be sent by the data provider to the source organization in a way of initiating the data transaction request by the data provider.

In some embodiments, the target data may be sent to the source organization when the data provider has a data sharing requirement, and the source organization may initiate the sharing transaction request to the blockchain network based on the target data obtained.

In some examples, the source organization or the data provider may initiate the sharing transaction request including the target data to the blockchain network. Correspondingly, the blockchain node may receive and process the sharing transaction request to perform chaining operation on the target data for storing the target data.

In some implementations, the blockchain node performs the chaining operation on the target data for storing the target data by calling a smart contract.

In some embodiments, the target data may be stored in a way of key-value pairs. A key domain may correspond to the source organization, and a value domain may be used to store target data stored by different source organizations. That is, different source organizations have different storage spaces of the blockchain, and the target data stored by the source organizations on the chain may be searched for based on an identifier of the source organization.

It may be understood that, the source organization may initiate the sharing transaction request for the target data of different data providers. In order to avoid data confusion, the target data may be generally stored by a dimension of the data providers.

Further, in order to avoid data leakage of the data providers, the target data of the data providers may be encrypted with keys of the data providers when the target data is stored by the dimension of the data providers.

In some implementations, using the key of the data provider to encrypt its target data may be implemented when performing the chaining operation on the target data for storing the target data. In some implementations, before the data provider provides the target data to the source organization, original content of the target data may be encrypted with the key of the data provider to obtain encrypted target data, and the encrypted target data may be provided to the source organization.

In some embodiments, in order to prevent the target data of the data provider from being leaked by organizations in the organization union other than the source organization and the target organization, the original content of the target data may be encrypted based on the key of the data provider and a key of the target organization.

In some implementations, encrypting the original content of the target data based on the key of the data provider and the key of the target organization may be implemented when the chaining operation is performed on the target data for storing the target data. In some implementations, before the data provider provides the data to the source organization, the original content may be encrypted based on the key of the data provider and the key of the target organization to obtain encrypted target data, and the encrypted target data may be provided to the source organization.

At block S102, a sharing smart contract is called to generate a sharing certificate including an identifier of the source organization, an identifier of the target organization and a storage identifier of the target data, and the chaining operation is performed on the sharing certificate for storing the sharing certificate.

The identifier of the source organization may be a name of the source organization or a blockchain account of the source organization in the blockchain network. The identifier of the target organization may be a name of the target organization or a blockchain account of the target organization in the blockchain network.

The storage identifier of the target data is used to access the target data of the data provider in a storage space, to which the source organization belongs, of the blockchain.

After the chaining operation is performed on the target data for storing the target data, the sharing smart contract may be associated and called to generate the sharing certificate including the identifier of the source organization, the identifier of the target organization, and the storage identifier of the target data. The chaining operation may be performed on the sharing certificate for storing the sharing certificate.

The sharing certificate may be used as a certification material for data sharing between the source organization and the target organization, and may also be used as a window for accessing the target data by the target organization.

In some implementations, the chaining operation may be performed on the target data for storing the target data, by calling a storage smart contract. The sharing certificate including the identifier of the source organization, the identifier of the target organization, and the storage identifier of the target data may be generated by calling the sharing smart contract. The chaining operation may be performed on the sharing certificate for storing the sharing certificate.

Data generated during calling different smart contracts may be managed by a set data storage model to form data sets, and the data sets of different smart contracts do not overlap with each other. Therefore, there is a certain reading barrier (difficulty) to read data from different data sets to which different smart contracts belong. In order to simplify the reading barrier (difficulty) of reading the target data and the sharing certificate during sharing the target data, one smart contract may be generally called to perform the chaining operation on the target data for storing the target data, and to generate the sharing certificate and perform the chaining operation on the sharing certificate for storing the sharing certificate.

Therefore, in some implementations, a data storage interface of the sharing smart contract may be called to perform the chaining operation on the target data for storing the target data. A sharing certification generating interface of the sharing smart contract may be called to generate the sharing certificate including the identifier of the source organization, the identifier of the target organization, and the storage identifier of the target data and to perform the chaining operation on the sharing certificate for storing the sharing certificate.

At block S103, the target data is shared with the target organization based on the sharing certificate.

In some examples, a storage space of the blockchain for storing the target data may be determined for the source organization based on the identifier of the source organization in the sharing certificate. The target data of the data provider may be obtained from the storage space, corresponding to the source organization, in the blockchain based on the storage identifier of the target data.

In some embodiments, the target organization may initiate a data obtaining transaction request including the sharing certificate when the target data is shared. The blockchain node may respond to the data obtaining transaction request initiated by the target organization, to obtain the target data based on the storage identifier of the target data and the identifier of the source organization in the sharing certificate and feed the target data back to the target organization.

In some examples, the target data may be obtained based on the storage identifier of the target data and the identifier of the source organization in the sharing certificate in a way of calling a data obtaining smart contract. The obtained target data may be fed back to the target organization.

It may be understood that, in order to improve the data obtaining efficiency, the target data may be obtained by calling a data obtaining interface in the sharing smart contract based on the storage identifier of the target data and the identifier of the source organization in the sharing certificate. The obtained target data may be fed back to the target organization.

The sharing certificate included in the data obtaining transaction request may be provided offline by the data provider to the target organization. In some examples, the target organization may search for the sharing certificate in the blockchain after learning that the data provider shares the target data.

In some implementations, in a case where the target data stored on the chain is data that is encrypted with the key of the data provider, after the target organization obtains the target data, the key of the data provider may be employed to decrypt the target data correspondingly. In some examples, the key for encrypting the target data may be a private key of the data provider, and the key for decrypting the target data may be a public key of the data provider.

In some implementations, in a case where the target data stored on the chain is data that is encrypted with the key of the data provider and the key of the target organization, after the target organization obtains the target data, the key of the data provider and the key of the target organization may be employed to decrypt the obtained target data correspondingly. In some examples, the key for encrypting the target data may be a symmetric encryption key determined by employing a preset encryption algorithm based on the private key of the data provider and the public key of the target organization. The key for decrypting the target data may be a symmetric encryption key determined by employing a preset encryption algorithm based on the public key of the data provider and the private key of the target organization. The preset encryption algorithm may be at least one encryption algorithm in the related art.

With embodiments of the disclosure, in response to the sharing transaction request including the target data of the data provider, the blockchain node may perform the chaining operation on the target data for storing the target data. The sharing smart contract may be called to generate the sharing certificate including the identifier of the source organization, the identifier of the target organization and the storage identifier of the target data. The chaining operation may be performed on the sharing certificate for storing the sharing certificate. The target data may be shared with the target organization based on the sharing certificate. With the above technical solution, the chaining operation is performed on the sharing certificate for storing the sharing certificate by calling the sharing smart contract, such that the target organization may obtain the target data based on the sharing certificate stored on the chain, thereby realizing data sharing of the target data between the source organization and the target organization. There is no need to issue or confirm a paper certificate to get an authorization and permission, thereby improving the data sharing efficiency. Meanwhile, the sharing certificate may be generated by calling the sharing smart contract, thereby reducing an impact of human operations on the accuracy of the sharing certificate and improving the accuracy of the sharing certificate.

FIG. 2 is a flowchart illustrating a method for sharing data based on a blockchain network according to embodiments of the disclosure.

Further, the operation "sharing the target data with the target organization based on the sharing certificate" may include "obtaining the target data based on the storage identifier of the target data and the identifier of the source organization in the sharing certificate and feeding the target data back to the target organization, in response to a data obtaining transaction request including the sharing certificate and sent by the target organization", to improve a sharing mechanism for sharing the target data the source organization with the target organization.

The method for sharing data based on a blockchain network as illustrated in FIG. 2 may include the following.

At block S201, in response to a sharing transaction request including target data of a data provider, a chaining operation is performed on the target data for storing the target data.

At block S202, a sharing smart contract is called, to generate a sharing certificate including an identifier of a source organization, an identifier of a target organization and a storage identifier of the target data, and the chaining operation is performed on the sharing certificate to store the sharing certificate.

In order to facilitate searching for and obtaining the sharing certificate, a storage identifier of the sharing certificate may be sent to at least one of the source organization, the data provider and the target organization after the chaining operation is performed on the sharing certificate to store the sharing certificate. The target organization may subsequently search for and obtain the sharing certificate based on the storage identifier of the sharing certificate, and search for the target data based on the storage identifier of the target data in the sharing certificate.

In some implementations, in order to improve the accuracy and security of the storage identifier of the sharing certificate, a to-be-processed queue may be set in the blockchain in advance correspondingly for each organization to store sharing events that are not processed yet. In some examples, the storage identifier of the sharing certificate may be sent to a to-be-processed queue of the target organization after the chaining operation is performed on the sharing certificate to store the sharing certificate, such that the target organization may obtain the sharing certificate based on the storage identifier of the sharing certificate in the to-be-processed queue.

In some implementations of the disclosure, the sharing certificate may also include a digital digest of the target data.

In some examples, when calling the sharing smart contract, and before generating the sharing certificate including the identifier of the source organization, the identifier of the target organization and the storage identifier of the target data, the method may further include encrypting the target data by a preset encoding function to obtain the digital digest of the target data.

Encoding different pieces of target data with the set encoding function may obtain different digital digests. Therefore, the digital digest may be used as a unique identifier of the target data and used to verify the accuracy of the target data subsequently when the target data is obtained.

In some implementations of the disclosure, performing the chaining operation on the sharing certificate for storing the sharing certificate to ensure the validity of the sharing certificate when the sharing certificate is obtained may include: sending the sharing certificate to the source organization, to allow the source organization to sign the sharing certificate through a private key of the source organization; obtaining a digital signature of the sharing certificate from the source organization, associating the digital signature with the sharing certificate, and performing the chaining operation on the digital signature and the sharing certificate for storing the digital signature and the sharing certificate. The digital signature may be used to verify the validity of the sharing certificate.

In some examples, signing, by the source organization, the sharing certificate through the private key of the source organization may include signing the sharing certificate by employing a set signature algorithm based on the private key of the source organization to obtain the digital signature.

At block S203, the target data is obtained based on the storage identifier of the target data and the identifier of the source organization in the sharing certificate, and the target data is fed back to the target organization, in response to a data obtaining transaction request including the sharing certificate and sent by the target organization.

In some examples, the target organization may obtain the sharing certificate and initiate the data obtaining transaction request including the sharing certificate to the blockchain network. The blockchain node may respond to the data obtaining transaction request and determine the storage space in the blockchain and corresponding to the source organization based on the identifier of the source organization in the sharing certificate. The target data may be searched for and obtained from the storage space associated with the source organization based on the storage identifier of the target data, and the obtained target data may be fed back to the target organization. In this way, data sharing of the target data may be implemented between the source organization and the target organization.

In some embodiments, the target organization may obtain the sharing certificate based on the storage identifier of the sharing certificate received by the target organization. In some embodiments, the target organization may obtain the storage identifier of the sharing certificate offline from the data provider or from the source organization, and obtain the sharing certificate based on the obtained storage identifier of the sharing certificate. In some embodiments, the target organization may initiate a queue access transaction request to the blockchain network. The blockchain node may call a queue access smart contract to obtain the storage identifier of the sharing certificate from the to-be-processed queue of the target organization. The sharing certificate may be obtained based on the storage identifier of the sharing certificate and fed back to the target organization.

In some examples, obtaining the sharing certificate based on the storage identifier of the sharing certificate may include the following. In a case where the target organization is a node participating in the blockchain network, the sharing certificate may be obtained locally based on the storage identifier of the sharing certificate, or the sharing certificate may be obtained from the chain. In a case where the target organization participates in the blockchain network through a trusted node, the target organization may send the storage identifier of the sharing certificate to the trusted node, and the trusted node may obtain the sharing certificate locally or obtain the sharing certificate from the chain.

In some implementations of the disclosure, in a case where the sharing certificate includes the digital digest of the target data, obtaining and feeding the target data back to the target organization based on the storage identifier of the target data and the identifier of the source organization in the sharing certificate may include the following. The target data is searched for based on the storage identifier of the target data and the identifier of the source organization in the sharing certificate. Verification is performed on the found target data based on the digital digest. The target data is obtained and fed back to the target organization when the found target data passes the verification.

In detail, performing verification on the found target data based on the digital digest may include the following. Calculation is performed based on found target data by employing a set coding function to obtain the digital digest. The calculated digital digest is compared with the digital digest in the sharing certificate to obtain a comparison result. The accuracy of the found target data is verified based on the comparison result. Accordingly, the found target data passes the verification when the calculated digital digest is consistent with the digital digest in the sharing certificate. When the calculated digital digest is not consistent with the digital digest in the sharing certificate, the found target data fails to pass the verification.

It may be understood that, when the found target data passes the verification, it may be indicated that the target data to be obtained by the target organization is the same as the found target data. When the found target data fails to pass the verification, it may be indicated that the target data to be obtained by the target organization is different from the found target data.

In some implementations of the disclosure, when the sharing certificate has the digital signature associated therewith, obtaining the target data based on the storage identifier of the target data and the identifier of the source organization in the sharing certificate and feeding the target data back to the target organization may include the following. Verification is performed on the digital signature from the source organization based on the identifier of the source organization in the sharing certificate. The target data is obtained and fed back to the target organization based on the storage identifier of the target data and the identifier of the source organization in the sharing certificate when the digital signature passes the verification.

In detail, performing verification on the digital signature from the source organization based on the identifier of the source organization in the sharing certificate may include the following. The public key of the source organization is searched for based on the identifier of the source organization in the sharing certificate. The sharing certificate is signed based on the public key of the source organization by employing a set signature algorithm to obtain the digital signature. The obtained digital signature is compared with the digital signature associated with the sharing certificate to obtain a comparison result. The validity of the sharing certificate is verified based on the comparison result. Accordingly, when the obtained digital signature is consistent with the digital signature associated with the sharing certificate, the sharing certificate passes the verification. When the obtained digital signature is not consistent with the digital signature associated with the sharing certificate,, the sharing certificate fails to pass the verification.

It may be understood that, when the sharing certificate passes the verification, it may be indicated that an initiator of the sharing certificate is the same as a signer of the sharing certificate. Therefore, it may be determined that the sharing certificate is valid. When the sharing certificate fails to pass the verification, it may be indicated that the initiator of the sharing certificate may be different from the signer of the sharing certificate. Therefore, it may be determined that the sharing certificate is invalid.

With embodiments of the disclosure, sharing the target data with the target organization may be implemented by processing the data obtaining transaction request including the sharing certificate initiated by the target organization, thereby improving the sharing mechanism of the target data and providing a technical support for the source organization to share the target data with the target organization.

The technical solutions according to the above embodiments of the disclosure may be used in a condition where a patient is transferred between a source hospital and a target hospital in a medical association including multiple hospitals.

In detail, the organizations in the above embodiments are hospitals. The target data of the data provider is diagnosis and treatment data of patients. The sharing certificate is a referral certificate.

FIG. 3 is a flowchart illustrating a method for sharing data based on a blockchain network according to embodiments of the disclosure. Embodiments of the disclosure may be applicable to a condition where target data is shared between a source organization and a target organization in an organization union containing multiple organizations. The method may be executed by an apparatus for sharing data based on a blockchain network. The apparatus may be implemented by software and/or hardware and integrated in a source organization.

The method for sharing data based on a blockchain network as illustrated in FIG. 3 may include the following.

At block S301, target data of a data provider is obtained.

In some embodiments, the target data of the data provider may be original content of the target data. In some embodiments, the target data refers to target data that is obtained by encrypting the original content of the target data with a key of the data provider. Therefore, only an organization having the key of the data provider may obtain the original content of the target data when the target data is shared.

In some embodiments, in order to prevent the target data of the data provider from being leaked from organizations other than the source organization and the target organization in the organization union, the data provider may encrypt the original content based on the key of the data provider and a key of the target organization. The encrypted target data may be provided to the source organization.

In some embodiments, in order to simplify the operation of the data provider, the original content of the target data may be encrypted by the source organization, instead of the data provider, based on the key of the data provider to obtain the target data, or the original content of the target data may be encrypted with the key of the data provider and the key of the target organization to obtain the target data. The key of the data provider may be a private key of the data provider. The key of the target organization may be a public key of the target organization.

At block S302, a sharing transaction request including the target data is initiated, to allow a blockchain node to perform a chaining operation on the target data for storing the target data. A sharing certificate including an identifier of the source organization, an identifier of a target organization and a storage identifier of the target data is generated by calling a sharing smart contract. Chaining operation is performed on the sharing certificate for storing the sharing certificate.

The sharing certificate is configured to instruct the target organization to share the target data.

The source organization may initiate the sharing transaction request including the target data to the blockchain network. The blockchain node may respond to the sharing transaction request, perform the chaining operation on the target data for storing the target data, call the sharing smart contract to generate the sharing certificate including the identifier of the source organization, the identifier of the target organization and the storage identifier of the target data, and perform the chaining operation on the sharing certificate for storing the sharing certificate. Correspondingly, the source organization may share the target data with the target organization based on the sharing certificate.

In some examples, sharing the target data with the target organization based on the sharing certificate may include the following. The target organization initiates a data obtaining transaction request including the sharing certificate to the blockchain network. The blockchain node responds to the data obtaining transaction request to obtain the target data based on the storage identifier of the target data and the identifier of the source organization in the sharing certificate and feed the target data back to the target organization.

Based on the technical solutions of the above embodiments, in order to facilitate the verification on the validity of the sharing certificate, performing the chaining operation on the sharing certificate at the blockchain node for storing the sharing certificate may include the following. The sharing certificate is sent to the source organization. Accordingly, the source organization signs the sharing certificate sent by the blockchain node with the private key of the source organization to obtain a digital signature of the sharing certificate, and feeds the digital signature back to the blockchain node to allow the blockchain node to associate the digital signature with the sharing certificate, and perform the chaining operation on the digital signature and the sharing certificate for storing the digital signature and the sharing certificate.

In some examples, signing, by the source organization, the sharing certificate with the private key of the source organization may include signing the sharing certificate by employing a set signature algorithm based on the private key of source organization to obtain the digital signature.

It may be understood that, in a case that the sharing certificate contains the digital signature from the source organization, the validity of the sharing certificate may be verified when the blockchain node shares the target data with the target organization based on the sharing certificate.

In some examples, obtaining the target data based on the storage identifier of the target data and the identifier of the source organization in the sharing certificate and feeding the target data back to the target organization when the sharing certificate has the digital signature associated therewith may include the following. Verification is performed on the digital signature of the source organization based on the identifier of the source organization in the sharing certificate. When the digital signature passes the verification, the target data is obtained based on the storage identifier of the target data and the identifier of the source organization in the sharing certificate, and the target data is fed back to the target organization.

In detail, performing verification on the digital signature of the source organization based on the identifier of the source organization in the sharing certificate may include the following. The public key of the source organization is searched for based on the identifier of the source organization in the sharing certificate. The sharing certificate is signed by employing the set signature algorithm based on the public key of the source organization to obtain the digital signature. The obtained digital signature is compared with the digital signature associated with the sharing certificate, to obtain a comparison result. The validity of the sharing certificate is verified based on the comparison result. Correspondingly, when the obtained digital signature is consistent with the digital signature associated with the sharing certificate, the obtained digital signature passes the verification. When the obtained digital signature is not consistent with the digital signature associated with the sharing certificate, the obtained digital signature fails to pass the verification.

It may be understood that, when the obtained digital signature passes the verification, it may be indicated that an initiator of the sharing certificate is the same as a signer of the sharing certificate. Therefore, it may be determined that the sharing certificate is valid. When the obtained digital signature fails to pass the verification, it may be indicated that the initiator of the sharing certificate is different from the signer of the sharing certificate. Therefore, it may be determined that the sharing certificate is invalid.

The technical solution according to any of the above embodiments of the disclosure may be specifically used in a case where a patient is transferred between a source hospital and a target hospital in a medical association including multiple hospitals.

In detail, the organizations mentioned in embodiments may be hospitals. The target data of the data provider may be diagnosis and treatment data of patients. The sharing certificate may be a referral certificate.

With embodiments of the disclosure, the source organization device may obtain the target data of the data provider and initiate the sharing transaction request including the target data, such that the blockchain node may perform the chaining operation on the target data for storing the target data, call the sharing smart contract to generate the sharing certificate including the identifier of the source organization, the identifier of the target organization and the storage identifier of the target data, and perform the chaining operation on the sharing certificate for sharing the sharing certificate. The sharing certificate may be used to instruct the target organization to share the target data. With the above technical solution, the chaining operation may be performed on the sharing certificate for storing the sharing certificate by calling the sharing smart contract, such that the target organization may obtain the target data based on the sharing certificate stored on the chain. In this way, data sharing of the target data may be implemented between the source organization and the target organization, without issuing or confirming a paper certificate to get an authorization and permission, thereby improving the data sharing efficiency. Meanwhile, the sharing certificate may be generated by calling the sharing smart contract, thereby reducing an impact of human operations on the accuracy of the sharing certificate and improving the accuracy of the sharing certificate.

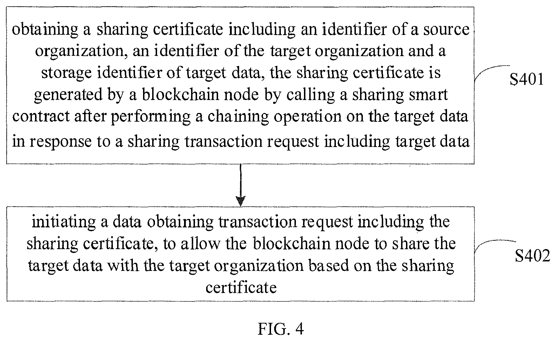

FIG. 4 is a flowchart illustrating a method for sharing data based on a blockchain network according to embodiments of the disclosure. Embodiments of the disclosure may be applicable to a condition where target data is shared between a source organization and a target organization in an organization union containing multiple organizations. The method may be executed by an apparatus for sharing data based on a blockchain network. The apparatus may be implemented by software and/or hardware and integrated in the source organization.

The method for sharing data based on a blockchain network as illustrated in FIG. 4 may include the following.

At block S401, a sharing certificate including an identifier of a source organization, an identifier of the target organization and a storage identifier of the target data is obtained. The sharing certificate may be generated by a blockchain node by calling a sharing contract after performing a chaining operation on the target data for storing the target data in response to a sharing transaction request including the target data.

A storage identification of the sharing certificate may be a storage identification for performing the chaining operation on the sharing certificate for storing the sharing certificate after the sharing certificate is generated by calling the sharing smart contract.

In some embodiments, the target organization may obtain the sharing certificate based on a received storage identifier of the sharing certificate. In some embodiments, the target organization may obtain the storage identifier of the sharing certificate offline from the data provider or from the source organization and obtain the sharing certificate based on the obtained storage identifier of the sharing certificate. In some embodiments, the target organization may initiate a queue access transaction request to the blockchain network, to allow the blockchain node to obtain the storage identifier of the sharing certificate from a to-be-processed queue of a chain of the target organization, and obtain the sharing certificate based on the storage identifier of the sharing certificate, and feed the sharing certificate back to the target organization.

In some examples, obtaining the sharing certificate based on the storage identifier of the sharing certificate may include the following. In a case where the target organization is a node participating in the blockchain network, the target organization may obtain the sharing certificate locally based on the storage identifier of the sharing certificate, or obtain the sharing certificate from the chain. In a case where the target organization participates in the blockchain network through a trusted node, the storage identifier of the sharing certificate may be sent to a trusted node, and the trusted node may obtain the sharing certificate locally or obtain the sharing certificate from the chain.

In some implementations of the disclosure, in the subsequent obtaining of the sharing certificate, in order to ensure the validity of the sharing certificate, the source organization may sign the sharing certificate when the sharing smart contract is called to generate the sharing certificate and the chaining operation is performed on the sharing certificate for storing the sharing certificate. In some examples, the sharing certificate may be sent to the source organization such that the source organization may sign the sharing certificate with a private key of the source organization. The digital signature of the sharing certificate may be obtained from the source organization, and the digital signature may be associated with the sharing certificate. The chaining operation may be performed on the digital signature and the sharing certificate for storing the digital signature and the sharing certificate. The digital signature may be used to verify the validity of the sharing certificate.

In some examples, signing, by the source organization, the sharing certificate with the private key of the source organization may include the following. The sharing certificate is signed by employing a set signature algorithm based on the private key of the source organization to obtain the digital signature.

At block S402, a data obtaining transaction request including the sharing certificate is initiated, to allow the blockchain node to share the target data with the target organization based on the sharing certificate.

In some examples, the target organization may initiate the data obtaining transaction request including the sharing certificate to the blockchain network. The blockchain node may respond to the data obtaining transaction request and share the target data with the target organization based on the sharing certificate.

In some embodiments, sharing the target data with the target organization based on the sharing certificate may include the following. A storage space in the blockchain for storing the target data is determined for the source organization based on the identifier of the source organization in the sharing certificate. The target data of the data provider is obtained based on the storage identifier of the target data from the storage space in the blockchain and corresponding to the source organization.

In some embodiments, the target organization may initiate the data obtaining transaction request including the sharing certificate when the target data is shared. The blockchain node may respond to the data obtaining transaction request initiated by the target organization to obtain the target data based on the storage identifier of the target data and the identifier of the source organization in the sharing certificate and feed the obtained target data back to the target organization.

In some examples, the target data may be obtained based on the storage identifier of the target data and the identifier of the source organization in the sharing certificate in a way of calling a data obtaining smart contract, and the obtained target data may be fed back to the target organization.

In some implementations of the disclosure, in a case where the sharing certificate has the digital signature associated therewith, in response to the data obtaining transaction request, obtaining, by the blockchain node, the target data based on the storage identifier of the target data and the identifier of the source organization in the sharing certificate and feeding the target data back to the target organization may include the following. Verification is performed on the digital signature from the source organization based on the identifier of the source organization in the sharing certificate. When the digital signature passes the verification, the target data is obtained based on the storage identifier of the target data and the identifier of the source organization in the sharing certificate and fed back to the target organization.

In detail, performing verification on the digital signature of the source organization based on the identifier of the source organization in the sharing certificate may include the following. A public key of the source organization may be searched for based on the identifier of the source organization in the sharing certificate. The sharing certificate is signed by employing the set signature algorithm based on the public key of the source organization to obtain the digital signature. The obtained digital signature is compared with the digital signature associated with the sharing certificate to obtain a comparison result. The validity of the sharing certificate is verified based on the comparison result. Correspondingly, the sharing certificate passes the verification when the obtained digital signature is consistent with the digital signature associated with the sharing certificate. When the obtained digital signature is not consistent with the digital signature associated with the sharing certificate, the sharing certificate fails to pass the verification.

It may be understood that, when the sharing certificate passes the verification, it may be indicated that an initiator of the sharing certificate is the same as a signer of the sharing certificate. Therefore, it may be determined that the sharing certificate is valid. When the sharing certificate fails to pass the verification, it may be indicated that the initiator of the sharing certificate is different from the signer of the sharing certificate. Therefore, it may be determined that the sharing certificate is invalid.

It should be noted that, in a case that the target data associated with the sharing data is encrypted data, the target data may be decrypted by the target organization after the blockchain node shares the target data with the target organization based on the sharing certificate in response to the initiated data obtaining transaction request including the sharing certificate.

In some examples, when the target data stored on the chain is data that is encrypted with the key of the data provider, the key of the data provider may be used to decrypt the target data after the target organization obtains the target data. For example, the key for encrypting the target data may be the private key of the data provider, and the key for decrypting the target data may be the public key of the data provider.

In some examples, when the target data stored on the chain is data that is encrypted with the key of the data provider and the key of the target organization, the target data may be decrypted by employing the key of the data provider and the key of the target organization after the target organization obtains the target data. For example, the key for encrypting the target data may be a symmetric encryption key determined by employing a set encryption algorithm based on the private key of the data provider and the public key of the target organization. The key for decrypting the target data may be a symmetric key determined by employing the set encryption algorithm based on the public key of the data provider and the private key of the target organization. The set encryption algorithm may be at least one encryption algorithm in the related art.

With embodiments of the disclosure, the target organization device may obtain the sharing certificate including the identifier of the source organization, the identifier of the target organization and the storage identifier of the target data. The sharing certificate may be generated by the blockchain node by calling the sharing smart contract after performing the chaining operation on the target data for storing the target data in response to the sharing transaction request including the target data. The data obtaining transaction request including the sharing certificate may be initiated, to allow the blockchain node to share the target data with the target organization based on the sharing certificate. With the above technical solution, the chaining operation is performed on the sharing certificate for storing the sharing certificate by calling the sharing smart contract, to allow the target organization to obtain the target data based on the sharing certificate stored on the chain. In this way, data sharing of the target data may be realized between the source organization and the target organization, without issuing or confirming a paper certificate to get an authorization and permission, thereby improving the data sharing efficiency. Meanwhile, the sharing certificate is generated by calling the sharing smart contract, thereby reducing the impact of human operations on the accuracy of the sharing certificate and improving the accuracy of the sharing certificate.

The technical solution according to any of the above embodiments of the disclosure may be specifically used in a case where a patient is transferred between a source hospital and a target hospital in a medical association including multiple hospitals.

In detail, the organizations mentioned in embodiments may be hospitals. The target data of the data provider may be diagnosis and treatment data of patients. The sharing certificate may be a referral certificate.

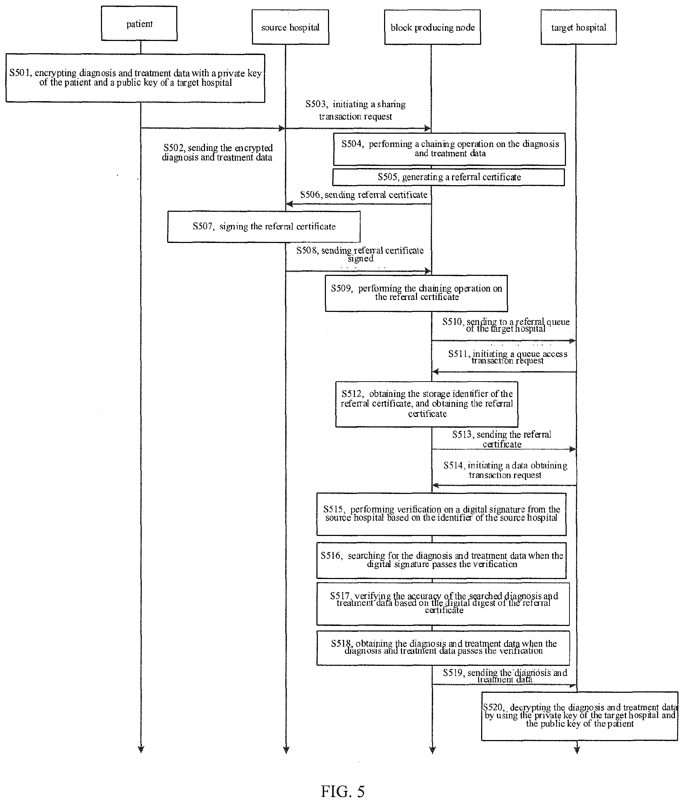

FIG. 5 is a flowchart illustrating a method for sharing data based on a blockchain network according to embodiments of the disclosure.

The method for sharing data based on a blockchain network as illustrated in FIG. 5 may include the following.

At block S501, diagnosis and treatment data may be encrypted with a private key of the patient and a public key of a target hospital by a patient.

In detail, an encryption key may be generated through an elliptic curve Diffie-Hellman (ECDH) algorithm by using the private key of the patient and the public key of the target hospital. The diagnosis and treatment data may be encrypted by employing the encryption key to obtain encrypted diagnosis and treatment data.

At block S502, the patient may send the encrypted diagnosis and treatment data to a source hospital.

At block S503, the source hospital may initiate a sharing transaction request including the diagnosis and treatment data.

At block S504, a block producing node may call a data storage interface of a sharing smart contract and perform a chaining operation on the diagnosis and treatment data for storing the diagnosis and treatment data.

Corresponding storage spaces in the blockchain network may be set for different hospitals. The diagnosis and treatment data may be stored in a key-value way. In order to distinguish the diagnosis and treatment data of different patients, the diagnosis and treatment data may be stored based on a dimension of patients.

At block S505, a referral certificate generating interface of the sharing smart contract may be called, to calculate a digital digest of the diagnosis and treatment data and generate a referral certificate including the source hospital, the target hospital, a storage identifier of the diagnosis and treatment data and the digital digest.

At block S506, the referral certificate may be sent to the source hospital.

At block S507, the source hospital may digitally sign the referral certificate by employing a private key of the source hospital.

At block S508, the referral certificate that is digitally signed may be fed back to the block producing node.

At block S509, the block producing node may perform the chaining operation on the referral certificate that is digitally signed for storing the referral certificate that is digitally signed.

At block S510, a storage identifier of the referral certificate may be sent to a referral queue of the target hospital.

Referral queues may be set in the blockchain network for different hospitals. The storage identifier of the referral certificate corresponding to a referral event may be added to the referral queue of the corresponding target hospital when the patient is referred between the hospitals included in the medical association.

It may be understood that, after the patient is successfully transferred, the storage identifier of the referral certificate corresponding to the referral event may be removed from the referral queue.

At block S511, the target hospital may initiate a queue access transaction request.

At block S512, the block producing node may call a queue access interface of the sharing smart contract to obtain the storage identifier of the referral certificate from the referral queue and obtain the referral certificate based on the storage identifier of the referral certificate.

At block S513, the referral certificate may be sent to the target hospital.

At block S514, the target hospital may initiate a data obtaining transaction request including the referral certificate.

At block S515, the block producing node may call a data obtaining interface of the sharing smart contract to perform verification on the validity of a digital signature from the source hospital based on the identifier of the source hospital in the referral certificate.

At block S516, the diagnosis and treatment data may be searched for based on the storage identifier of the diagnosis and treatment data when the digital signature passes the verification.

At block S517, the accuracy of the found diagnosis and treatment data may be verified based on the digital digest of the referral certificate.

At block S518, the diagnosis and treatment data may be obtained when the found diagnosis and treatment data passes the verification.

At block S519, the diagnosis and treatment data may be sent to the target hospital.

At block S520, the target hospital may decrypt the diagnosis and treatment data by using the private key of the target hospital and the public key of the patient.

In detail, the target hospital may use the private key of the target hospital and the public key of patient to generate a decryption key through the ECDH algorithm, and use the decryption key to decrypt diagnosis and treatment data.

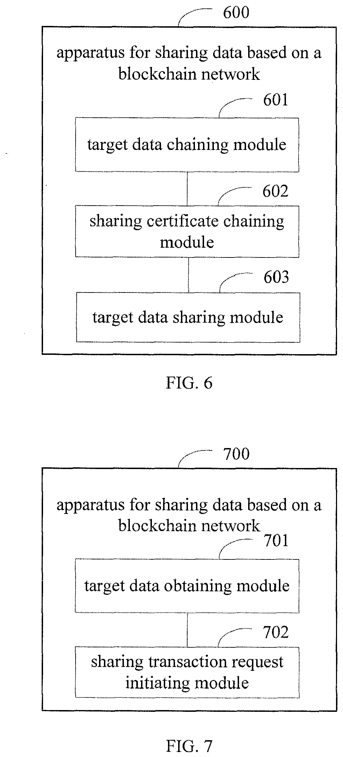

FIG. 6 is a block diagram illustrating an apparatus for sharing data based on a blockchain network according to embodiments of the disclosure. Embodiments of the disclosure are applicable to a condition where target data is shared between a source organization and a target organization in an organization union containing multiple organizations. The apparatus is implemented by software and/or hardware, and configured in an electronic device carrying a blockchain node.

The apparatus 600 for sharing the data based on the blockchain network illustrated in FIG. 6 includes: a target data chaining module 601, a sharing certificate chaining module 602, and a target data sharing module 603.

The target data chaining module 601 is configured to, in response to a sharing transaction request including target data of a data provider, perform a chaining operation on the target data for storing the target data.

The sharing certificate chaining module 602 is configured to call a sharing smart contract, to generate a sharing certificate including an identifier of a source organization, an identifier of a target organization and a storage identifier of the target data, and perform the chaining operation on the sharing certificate for storing the sharing certificate.

The target data sharing module 603 is configured to share the target data with the target organization based on the sharing certificate.

With embodiments of the disclosure, the target data chaining module is configured to, in response to the sharing transaction request including the target data of the data provider, perform the chaining operation on the target data for storing the target data. The sharing certificate chaining module is configured to call the sharing smart contract, to generate the sharing certificate including the identifier of the source organization, the identifier of the target organization and the storage identifier of the target data, and perform the chaining operation on the sharing certificate for storing the sharing certificate. The target data sharing module is configured to share the target data with the target organization based on the sharing certificate. With the above technical solution, the chaining operation is performed on the sharing certificate for storing the sharing certificate by calling the sharing smart contract, such that the target organization obtains the target data based on the sharing certificate stored on the chain, thereby realizing to share the target data between the source organization and the target organization. There is no need to issue and confirm the paper certificate for authorization, improving the data sharing efficiency. Meanwhile, the sharing certificate is generated by calling the sharing smart contract, thereby reducing the impact of human operations on the accuracy of the sharing certificate, and improving the accuracy of the sharing certificate.

Further, the target data sharing module 603 is configured to: obtain the target data based on the storage identifier of the target data and the identifier of the source organization in the sharing certificate and feed the target data back to the target organization, in response to a data obtaining transaction request including the sharing certificate and sent by the target organization.

Further, the sharing certificate chaining module 602 is configured to: send a storage identifier of the sharing certificate to a to-be-processed queue of the target organization, to allow the target organization to obtain the sharing certificate based on the storage identifier of the sharing certificate in the to-be-processed queue before responding to the data obtaining transaction request including the sharing certificate and sent by the target organization

Further, the target data sharing module 603 is also configured to: call a queue access smart contract to obtain the storage identifier of the sharing certificate from the to-be-processed queue of the target organization in response to a queue access transaction request initiated by the target organization; and obtain the sharing certificate based on the storage identifier of the sharing certificate, and feed the sharing certificate back to the target organization.

Further, the sharing certificate also includes a digital digest of the target data.

Correspondingly, the target data sharing module 603 is also configured to: when obtaining the target data based on the storage identifier of the target data and the identifier of the source organization in the sharing certificate and feeding the target data back to the target organization, search for the target data based on the storage identifier of the target data and the identifier of the source organization in the sharing certificate; perform verification on the target data found based on the digital digest; and obtain the target data and feeding the target data back to the target organization when the target data passes the verification.

Further, the target data chaining module 601 is configured to: when performing the chaining operation on the sharing certificate for storing the sharing certificate, send the sharing certificate to the source organization, to allow the source organization to sign the sharing certificate through a private key of the source organization; and obtain a digital signature of the sharing certificate from the source organization, associate the digital signature with the sharing certificate, and perform the chaining operation on the digital signature and the sharing certificate for storing the digital signature and the sharing certificate.

Correspondingly, the target data sharing module 603 is also configured to: when obtaining the target data based on the storage identifier of the target data and the identifier of the source organization in the sharing certificate and feeding the target data back to the target organization, perform verification on the digital signature from the source organization based on the identifier of the source organization in the sharing certificate; and obtain the target data based on the storage identifier of the target data and the identifier of the source organization in the sharing certificate and feed the target data back to the target organization when the digital signature passes the verification.

Further, the target data chaining module 601 is configured to: call a data storage interface of the sharing smart contract to perform the chaining operation on the target data for storing the target data when performing the chaining operation on the target data for storing the target data.

Further, the sharing certificate chaining module 602 is configured to: call a sharing certificate generating interface of the sharing smart contract to generate the sharing certificate including the identifier of the source organization, the identifier of the target organization and the storage identifier of the target data, and perform the chaining operation on the sharing certificate for storing the sharing certificate when calling the sharing smart contract to generate the sharing certificate comprising the identifier of the source organization, the identifier of the target organization and the storage identifier of the target data and performing the chaining operation on the sharing certificate for storing the sharing certificate.

Further, the target data is obtained by encrypting original content based on a key of the data provider and a key of the target organization.

Further, the organization is a hospital, the target data of the data provider is diagnosis and treatment data of patients, and the sharing certificate is a referral certificate.

The above apparatus for sharing the data based on the blockchain network may execute the method for sharing the data based on the blockchain network according to any one of embodiments of the disclosure, and has corresponding functional modules and beneficial effects for executing the method for sharing the data based on the blockchain network.

FIG. 7 is a block diagram illustrating an apparatus for sharing data based on a blockchain network according to embodiments of the disclosure. Embodiments of the disclosure are applicable to a condition where target data is shared between a source organization and a target organization in an organization union containing multiple organizations. The apparatus is implemented by software and/or hardware, and configured in the source organization.

The apparatus 700 for sharing the data based on the blockchain network illustrated in FIG. 7 includes: a target data obtaining module 701 and a sharing transaction request initiating module 702.

The target data obtaining module 701 is configured to obtain target data of a data provider.

The sharing transaction request initiating module 702 is configured to initiate a sharing transaction request including the target data, to allow a blockchain node to perform a chaining operation on the target data for storing the target data , generate a sharing certificate including an identifier of a source organization, an identifier of a target organization and a storage identifier of the target data by calling a sharing smart contract, and perform a chaining operation on the sharing certificate for storing the sharing certificate.

The sharing certificate is configured to indicate the target organization to share the target data.

With embodiments of the disclosure, the target data obtaining module is configured to obtain the target data of the data provider. The sharing transaction request initiating module is configured to initiate the sharing transaction request including the target data, to allow the blockchain node to perform the chaining operation on the target data for storing the target data, generate the sharing certificate including the identifier of the source organization, the identifier of the target organization and the storage identifier of the target data by calling the sharing smart contract, and perform the chaining operation on the sharing certificate for storing the sharing certificate. The sharing certificate is configured to indicate the target organization to share the target data. With the above technical solution, the chaining operation is performed on the sharing certificate for storing the sharing certificate by calling the sharing smart contract, such that the target organization obtains the target data based on the sharing certificate stored in the chain. In this way, it is implemented that the target data is shared between the source organization and the target organization, and there is no need to issue and confirm the paper certificate for authorization, which improves the data sharing efficiency. Meanwhile, the sharing certificate is generated by calling the sharing smart contract, thereby reducing the impact of human operations on the accuracy of the sharing certificate, and improving the accuracy of the sharing certificate.

Further, the apparatus also includes a signing module. The signing module is configured to: sign the sharing certificate sent by the blockchain node through a private key of the source organization to obtain a digital signature of the sharing certificate; and feed the digital signature back to the blockchain node, to allow the blockchain node to associate the digital signature with the sharing certificate, and perform the chaining operation on the digital signature and the sharing certificate for storing the digital signature and the sharing certificate.

Further, the target data obtaining module 701 is configured to: encrypt original content based on a key of the data provider and a key of the target organization to obtain the target data.

The above apparatus for sharing the data based on the blockchain network may execute the method for sharing the data based on the blockchain network according to any one of embodiments of the disclosure, and has corresponding functional modules and beneficial effects for executing the method for sharing the data based on the blockchain network.

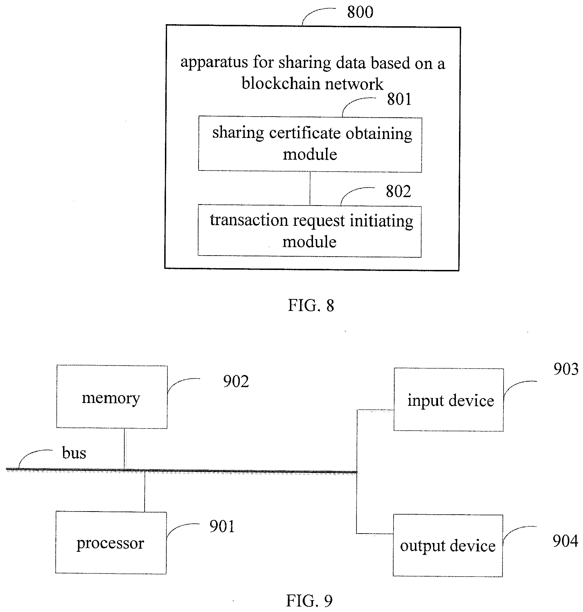

FIG. 8 is a block diagram illustrating an apparatus for sharing data based on a blockchain network according to embodiments of the disclosure. Embodiments of the disclosure are applicable to a condition where target data is shared between a source organization and a target organization in an organization union containing multiple organizations. The method is executed by an apparatus for sharing data based on a blockchain network. The apparatus is implemented by software and/or hardware, and configured in the target organization.

The apparatus 800 for sharing the data based on the blockchain network illustrated in FIG. 8 includes: a sharing certificate obtaining module 801 and a transaction request initiating module 802.

The sharing certificate obtaining module 801 is configured to obtain a sharing certificate including an identifier of a source organization, an identifier of the target organization and a storage identifier of target data. The sharing certificate is generated by a blockchain node after performing a chaining operation on the target data for storing the target data and calling a sharing smart contract in response to a sharing transaction request comprising the target data.

The transaction request initiating module 802 is configured to initiate a data obtaining transaction request including the sharing certificate, to allow the blockchain node to share the target data with the target organization based on the sharing certificate.

With embodiments of the disclosure, the sharing certificate obtaining module is configured to obtain the sharing certificate including the identifier of the source organization, the identifier of the target organization and the storage identifier of the target data. The sharing certificate is generated by the blockchain node after performing the chaining operation on the target data for storing the target data and calling the sharing smart contract in response to the sharing transaction request comprising the target data. The transaction request initiating module is configured to initiate the data obtaining transaction request comprising the sharing certificate, to allow the blockchain node to share the target data with the target organization based on the sharing certificate. With the above technical solution, the chaining operation is performed on the sharing certificate for storing the sharing certificate by calling the sharing smart contract, such that the target organization obtains the target data based on the sharing certificate stored in the chain. In this way, it is implemented that the target data is shared between the source organization and the target organization, and there is no need to issue and confirm the paper certificate for authorization, which improves the data sharing efficiency. Meanwhile, the sharing certificate is generated by calling the sharing smart contract, thereby reducing the impact of human operations on the accuracy of the sharing certificate, and improving the accuracy of the sharing certificate.

Further, the sharing certificate obtaining module 801 is configured to: initiate a queue access transaction request, to allow the blockchain node to obtain a storage identifier of the sharing certificate from a to-be-processed queue of a chain of the target organization, obtain the sharing certificate based on the storage identifier of the sharing certificate, and feed the sharing certificate back to the target organization.

Further, the apparatus also includes a decrypting module. The decrypting module is configured to decrypt the target data based on a key of the data provider and a key of the target organization.

The above apparatus for sharing the data based on the blockchain network may execute the method for sharing the data based on the blockchain network according to any one of embodiments of the disclosure, and has corresponding functional modules and beneficial effects for executing the method for sharing the data based on the blockchain network.

According to embodiments of the disclosure, the disclosure also provides an electronic device and a readable storage medium.

As illustrated in FIG. 9, FIG. 9 is a block diagram illustrating an electronic device capable of implementing a method for sharing data based on a blockchain network according to embodiments of the disclosure. The electronic device aims to represent various forms of digital computers, such as a laptop computer, a desktop computer, a workstation, a personal digital assistant, a server, a blade server, a mainframe computer and other suitable computer. The electronic device may also represent various forms of mobile devices, such as personal digital processing, a cellular phone, a smart phone, a wearable device and other similar computing device. The components, connections and relationships of the components, and functions of the components illustrated herein are merely examples, and are not intended to limit the implementation of the disclosure described and/or claimed herein.

As illustrated in FIG. 9, the electronic device includes: one or more processors 901, a memory 902, and interfaces for connecting various components, including a high-speed interface and a low-speed interface. Various components are connected to each other via different buses, and may be mounted on a common main board or in other ways as required. The processor may process instructions executed within the electronic device, including instructions stored in or on the memory to display graphical information of the GUI (graphical user interface) on an external input/output device (such as a display device coupled to an interface). In other implementations, multiple processors and/or multiple buses may be used together with multiple memories if desired. Similarly, multiple electronic devices may be connected, and each device provides some necessary operations (for example, as a server array, a group of blade servers, or a multiprocessor system). In FIG. 9, a processor 901 is taken as an example.

The memory 902 is a non-transitory computer readable storage medium provided by the disclosure. The memory is configured to store instructions executable by at least one processor, to enable the at least one processor to execute the method for sharing the data based on the blockchain network provided by the disclosure. The non-transitory computer readable storage medium provided by the disclosure is configured to store computer instructions. The computer instructions are configured to enable a computer to execute the method for sharing the data based on the blockchain network provided by the disclosure.

As the non-transitory computer readable storage medium, the memory 902 may be configured to store non-transitory software programs, non-transitory computer executable programs and modules, such as program instructions/module (such as the target data chaining module 601, the sharing certificate chaining module 602, and the target data sharing module 603 illustrated in FIG. 6; the target data obtaining module 701 and the sharing transaction request initiating module 702 illustrated in FIG. 7; or the sharing certificate obtaining module 801 and the transaction request initiating module 802 illustrated in FIG. 8) corresponding to the method for sharing the data based on the blockchain network according to embodiments of the disclosure. The processor 901 is configured to execute various functional applications and data processing of the server by operating non-transitory software programs, instructions and modules stored in the memory 902, that is, implements the method for sharing the data based on the blockchain network according to the above method embodiments.

The memory 902 may include a storage program region and a storage data region. The storage program region may store an application required by an operating system and at least one function. The storage data region may store data created according to predicted usage of the electronic device based on the semantic representation. In addition, the memory 902 may include a high-speed random access memory, and may also include a non-transitory memory, such as at least one disk memory device, a flash memory device, or other non-transitory solid-state memory device. In some embodiments, the memory 902 may optionally include memories remotely located to the processor 901, and these remote memories may be connected to the electronic device via a network. Examples of the above network include, but are not limited to, an Internet, an intranet, a local area network, a mobile communication network and combinations thereof.

The electronic device capable of implementing the method for sharing the data based on the blockchain network may also include: an input device 903 and an output device 904. The processor 901, the memory 902, the input device 903, and the output device 904 may be connected via a bus or in other means. In FIG. 9, the bus is taken as an example.

The input device 903 may receive inputted digital or character information, and generate key signal input related to user setting and function control of the electronic device capable of implementing the method for recognizing the entity word, such as a touch screen, a keypad, a mouse, a track pad, a touch pad, an indicator stick, one or more mouse buttons, a trackball, a joystick and other input device. The output device 904 may include a display device, an auxiliary lighting device (e.g., LED), a haptic feedback device (e.g., a vibration motor), and the like. The display device may include, but be not limited to, a liquid crystal display (LCD), a light emitting diode (LED) display, and a plasma display. In some embodiments, the display device may be the touch screen.

The various implementations of the system and technologies described herein may be implemented in a digital electronic circuit system, an integrated circuit system, an application specific ASIC (application specific integrated circuit), a computer hardware, a firmware, a software, and/or combinations thereof. These various implementations may include: being implemented in one or more computer programs. The one or more computer programs may be executed and/or interpreted on a programmable system including at least one programmable processor. The programmable processor may be a special purpose or general purpose programmable processor, may receive data and instructions from a storage system, at least one input device, and at least one output device, and may transmit data and the instructions to the storage system, the at least one input device, and the at least one output device.

These computing programs (also called programs, software, software applications, or codes) include machine instructions of programmable processors, and may be implemented by utilizing high-level procedures and/or object-oriented programming languages, and/or assembly/machine languages. As used herein, the terms "machine readable medium" and "computer readable medium" refer to any computer program product, device, and/or apparatus (such as, a magnetic disk, an optical disk, a memory, a programmable logic device (PLD)) for providing machine instructions and/or data to a programmable processor, including a machine readable medium that receives machine instructions as a machine readable signal. The term "machine readable signal" refers to any signal for providing the machine instructions and/or data to the programmable processor.

To provide interaction with a user, the system and technologies described herein may be implemented on a computer. The computer has a display device (such as, a CRT (cathode ray tube) or a LCD (liquid crystal display) monitor) for displaying information to the user, a keyboard and a pointing device (such as, a mouse or a trackball), through which the user may provide the input to the computer. Other types of devices may also be configured to provide interaction with the user. For example, the feedback provided to the user may be any form of sensory feedback (such as, visual feedback, auditory feedback, or tactile feedback), and the input from the user may be received in any form (including acoustic input, voice input or tactile input).

The system and technologies described herein may be implemented in a computing system including a background component (such as, a data server), a computing system including a middleware component (such as, an application server), or a computing system including a front-end component (such as, a user computer having a graphical user interface or a web browser through which the user may interact with embodiments of the system and technologies described herein), or a computing system including any combination of such background component, the middleware components and the front-end component. Components of the system may be connected to each other via digital data communication in any form or medium (such as, a communication network). Examples of the communication network include a local area network (LAN), a wide area networks (WAN), and the Internet.

The computer system may include a client and a server. The client and the server are generally remote from each other and generally interact via the communication network. A relationship between the client and the server is generated by computer programs operated on a corresponding computer and having a client-server relationship with each other.

With embodiments of the disclosure, in response to the sharing transaction request including the target data of the data provider, the chaining operation is performed on the target data for storing the target data. The sharing smart contract is called to generate the sharing certificate including the identifier of the source organization, the identifier of the target organization and the storage identifier of the target data. The chaining operation is performed on the sharing certificate for storing the sharing certificate. The target data is shared with the target organization based on the sharing certificate. With the above technical solution, the chaining operation is performed on the sharing certificate for storing the sharing certificate by calling the sharing smart contract, such that the target organization obtains the target data based on the sharing certificate stored on the chain, thereby realizing to share the target data between the source organization and the target organization. There is no need to issue and confirm the paper certificate for authorization, improving the data sharing efficiency. Meanwhile, the sharing certificate is generated by calling the sharing smart contract, thereby reducing the impact of human operations on the accuracy of the sharing certificate, and improving the accuracy of the sharing certificate.

It should be understood that, steps may be reordered, added or deleted by utilizing flows in the various forms illustrated above. For example, the steps described in the disclosure may be executed in parallel, sequentially or in different orders, so long as desired results of the technical solution disclosed in the disclosure may be achieved, there is no limitation here.

The above detailed implementations do not limit the protection scope of the disclosure. It should be understood by the skilled in the art that various modifications, combinations, sub-combinations and substitutions may be made based on design requirements and other factors. Any modification, equivalent substitution and improvement made within the spirit and the principle of the disclosure shall be included in the protection scope of disclosure.

## Claims

1. A method for sharing data based on a blockchain network, executed by a blockchain node in the blockchain network, the method comprising:
in response to a sharing transaction request comprising target data of a data provider, performing (S101; S201) a chaining operation on the target data for storing the target data;
calling (S102; S202) a sharing smart contract to generate a sharing certificate comprising an identifier of a source organization, an identifier of a target organization and a storage identifier of the target data, and performing the chaining operation on the sharing certificate for storing the sharing certificate; and
sharing (S103) the target data with the target organization based on the sharing certificate.

2. The method of claim 1, wherein sharing (S103) the target data with the target organization based on the sharing certificate comprises:
obtaining (S203) the target data based on the storage identifier of the target data and the identifier of the source organization in the sharing certificate and feeding the target data back to the target organization, in response to a data obtaining transaction request comprising the sharing certificate and sent by the target organization.

3. The method of claim 2, after performing the chaining operation on the sharing certificate for storing the sharing certificate and before responding to the data obtaining transaction request comprising the sharing certificate and sent by the target organization, further comprising:
sending a storage identifier of the sharing certificate to a to-be-processed queue of the target organization, to allow the target organization to obtain the sharing certificate based on the storage identifier of the sharing certificate in the to-be-processed queue.

4. The method of claim 3, further comprising:
calling a queue access smart contract to obtain the storage identifier of the sharing certificate from the to-be-processed queue of the target organization in response to a queue access transaction request initiated by the target organization; and
obtaining the sharing certificate based on the storage identifier of the sharing certificate, and feeding the sharing certificate back to the target organization.

5. The method of claim 2, wherein the sharing certificate further comprises a digital digest of the target data; and obtaining the target data based on the storage identifier of the target data and the identifier of the source organization in the sharing certificate and feeding the target data back to the target organization comprises:
searching for the target data based on the storage identifier of the target data and the identifier of the source organization in the sharing certificate;
performing verification on the target data found based on the digital digest; and
obtaining the target data and feeding the target data back to the target organization when the target data passes the verification.

6. The method of claim 2, wherein performing the chaining operation on the sharing certificate for storing the sharing certificate comprises:
sending the sharing certificate to the source organization, to allow the source organization to sign the sharing certificate through a private key of the source organization; and
obtaining a digital signature of the sharing certificate from the source organization, associating the digital signature with the sharing certificate, and performing the chaining operation on the digital signature and the sharing certificate for storing the digital signature and the sharing certificate;
wherein obtaining the target data based on the storage identifier of the target data and the identifier of the source organization in the sharing certificate and feeding the target data back to the target organization comprises:
performing verification on the digital signature from the source organization based on the identifier of the source organization in the sharing certificate; and
obtaining the target data based on the storage identifier of the target data and the identifier of the source organization in the sharing certificate and feeding the target data back to the target organization when the digital signature passes the verification.

7. The method of claim 1, wherein performing the chaining operation on the target data for storing the target data comprises:
calling a data storage interface of the sharing smart contract to perform the chaining operation on the target data for storing the target data; and
wherein calling the sharing smart contract to generate the sharing certificate comprising the identifier of the source organization, the identifier of the target organization and the storage identifier of the target data and performing the chaining operation on the sharing certificate comprises:
calling a sharing certificate generating interface of the sharing smart contract to generate the sharing certificate comprising the identifier of the source organization, the identifier of the target organization and the storage identifier of the target data and performing the chaining operation on the sharing certificate for storing the sharing certificate.

8. A method for sharing data based on a blockchain network, executed by a source organization, the method comprising:
obtaining (S301) target data of a data provider;
initiating (S302) a sharing transaction request comprising the target data, to allow a blockchain node to perform a chaining operation on the target data for storing the target data, generate a sharing certificate comprising an identifier of the source organization, an identifier of a target organization and a storage identifier of the target data by calling a sharing smart contract, and perform the chaining operation on the sharing certificate for storing the sharing certificate, wherein the sharing certificate is configured to indicate the target organization to share the target data.

9. The method of claim 8, further comprising:
signing the sharing certificate sent by the blockchain node through a private key of the source organization to obtain a digital signature of the sharing certificate; and
feeding the digital signature back to the blockchain node, to allow the blockchain node to associate the digital signature with the sharing certificate, and perform the chaining operation on the digital signature and the sharing certificate for storing the digital signature and the sharing certificate.

10. The method of claim 8, wherein obtaining (S301) the target data of the data provider comprises:
encrypting original content based on a key of the data provider and a key of the target organization to obtain the target data.

11. A method for sharing data based on a blockchain network, executed by a target organization, the method comprising:
obtaining (S401) a sharing certificate comprising an identifier of a source organization, an identifier of the target organization and a storage identifier of target data, wherein the sharing certificate is generated by a blockchain node by calling a sharing smart contract after performing a chaining operation on the target data for storing the target data in response to a sharing transaction request comprising the target data; and
initiating (S402) a data obtaining transaction request comprising the sharing certificate, to allow the blockchain node to share the target data with the target organization based on the sharing certificate.

12. The method of claim 11, wherein obtaining (S401) the sharing certificate comprising the identifier of the source organization, the identifier of the target organization and the storage identifier of the target data comprises:
initiating a queue access transaction request, to allow the blockchain node to obtain a storage identifier of the sharing certificate from a to-be-processed queue of a chain of the target organization, obtain the sharing certificate based on the storage identifier of the sharing certificate, and feed the sharing certificate back to the target organization.

13. The method of claim 11, further comprising:
decrypting the target data based on a key of the data provider and a key of the target organization.

14. An apparatus (600) for sharing data based on a blockchain network, integrated into a blockchain node, the apparatus comprising:
a target data chaining module (601), configured to, in response to a sharing transaction request comprising target data of a data provider, perform a chaining operation on the target data for storing the target data;
a sharing certificate chaining module (602), configured to call a sharing smart contract, to generate a sharing certificate comprising an identifier of a source organization, an identifier of a target organization and a storage identifier of the target data, and perform the chaining operation on the sharing certificate for storing the sharing certificate; and
a target data sharing module (603), configured to share the target data with the target organization based on the sharing certificate.

15. An electronic device, comprising:
at least one processor (91); and
a memory (92), communicatively coupled to the at least one processor (91),
wherein the memory (92) is configured to store instructions executable by the at least one processor (91), and when the instructions are executed by the at least one processor (91), the at least one processor (91) is caused to execute a method for sharing data based on a blockchain network according to any one of claims 1 to 13.

16. A non-transitory computer readable storage medium having computer instructions stored thereon, wherein the computer instructions are configured to cause a computer to execute a method for sharing data based on a blockchain network according to any one of claims 1 to 13.

17. A computer program product, containing computer programs when executed on a processor, cause the processor to perform a method for sharing data based on a blockchain network according to any one of claims 1 to 13.
